# EUROPEAN PATENT APPLICATION

(11) **EP 4 059 350 A1**
(43) Date of publication of application: **21.09.2022**
(21) Application number: 20887011.3
(22) Date of filing: 22.10.2020
(51) Int. Cl.: A23C 9/123

(54) **METHOD FOR PRODUCING FERMENTED MILK CONTAINING OLIGOSACCHARIDES**

(30) Priority: 11.11.2019 JP 2019204283
(71) Applicant: Kabushiki Kaisha Yakult Honsha, Tokyo, 105-8660 (JP)
(72) Inventor: NAKAMORI, Maiko, Tokyo (JP); IGARASHI, Shiori, Tokyo (JP); NIHEI, Daichi, Tokyo (JP)
(74) Representative: Blodig, Wolfgang
(86) International application number: PCT/JP2020/039662
(87) International publication number: WO 2021/095476

(57) **Abstract**

Using a method for producing fermented milk containing an oligosaccharide and a lactic acid bacterium as a living bacterium which is characterized by culturing the lactic acid bacterium in a medium containing the oligosaccharide and fermented milk produced by the production method, a high viable bacterial count can be maintained even during long-term preservation in the fermented milk containing the oligosaccharide and the lactic acid bacterium as a living bacterium.

## Description

### Technical Field

The present invention relates to a method for producing fermented milk containing an oligosaccharide and a lactic acid bacterium as a living bacterium.

### Background Art

Fermented milk containing a lactic acid bacterium has long been known, and it has been also known to add saccharides as syrup to add various effects and sweetness.

For example, fermented milk containing a living lactic acid bacterium and an oligosaccharide as a saccharide is known, and the fermented milk is known to have excellent intestine reachability (PTL 1).

It was found, however, that, although the fermented milk containing a living lactic acid bacterium and an oligosaccharide has excellent intestine reachability, a high viable bacterial count is more difficult to maintain during long-term preservation when the oligosaccharide is added as syrup compared to the case in which a saccharide other than oligosaccharides is added as syrup.

### Citation List

### Patent Literature

PTL 1: Patent No. JP 2571734

### Summary of Invention

### Technical Problem

Accordingly, an object of the present invention is to provide a technique which can maintain a high viable bacterial count even during long-term preservation in fermented milk containing an oligosaccharide and a lactic acid bacterium as a living bacterium.

### Solution to Problem

As a result of intensive studies to achieve the object, the present inventors have found that, when fermented milk containing an oligosaccharide and a lactic acid bacterium as a living bacterium is produced, the object can be achieved by adding the oligosaccharide to a medium for culturing the lactic acid bacterium, instead of adding the oligosaccharide as syrup as in the conventional method. The present invention has been thus completed.

That is, the present invention is a method for producing fermented milk containing an oligosaccharide and a lactic acid bacterium as a living bacterium which is characterized by culturing the lactic acid bacterium in a medium containing the oligosaccharide.

Moreover, the present invention is fermented milk produced by the method for producing fermented milk.

Furthermore, the present invention is a method for improving the viability of a lactic acid bacterium in fermented milk containing an oligosaccharide and the lactic acid bacterium as a living bacterium which is characterized by culturing the lactic acid bacterium in a medium containing the oligosaccharide.

### Advantageous Effects of Invention

Because the method for producing fermented milk of the present invention is a simple method in which an oligosaccharide is added to a medium for culturing a lactic acid bacterium, the existing facility can be used as it is without especially introducing a new facility or the like.

Fermented milk produced by the method for producing fermented milk of the present invention can not only maintain a high viable bacterial count during long-term preservation but also has improved flavor and an enhanced healthy image.

In particular, fermented milk obtained by the method for producing fermented milk of the present invention enables simultaneous intake of a probiotic material due to the lactic acid bacterium and a prebiotic material due to the oligosaccharide which increases the lactic acid bacterium in the intestines and thus is a so-called synbiotic material which further enhances the function of the probiotic to protect the health of the stomach.

### Description of Embodiments

The method for producing fermented milk of the present invention (referred to as "the production method of the present invention" below) is a method for producing fermented milk containing an oligosaccharide and a lactic acid bacterium as a living bacterium, and the lactic acid bacterium is cultured in a medium containing the oligosaccharide.

The oligosaccharide used in the production method of the present invention is not particularly limited, and examples thereof include maltooligosaccharides, fructooligosaccharides, galactooligosaccharides and the like. The oligosaccharide used in the present invention refers to transferred disaccharides or tri- and higher saccharides. Of the oligosaccharides, oligosaccharides containing galactose in the saccharide chain are preferable, and galactooligosaccharides are more preferable. In particular, galactooligosaccharides containing 4'-GL as the main component are preferable. Examples of the galactooligosaccharides include Oligomate 55N (manufactured by Yakult Pharmaceutical Industry Co., Ltd.: Brix sugar content of 75.0 to 76.0), which is a mixture of 56.4% galactooligosaccharides containing 4'-GL as the main component and 43% other saccharides, and the like.

The oligosaccharide is contained in the medium described below in an amount of 1.0 mass% (simply referred to as "%" below) or more, preferably 5.0% or more, more preferably 5 to 20%, particularly preferably 5 to 15%.

The medium used in the production method of the present invention is not particularly limited as long as the lactic acid bacterium can grow in the medium, and examples thereof include a mammalian milk medium composed of raw milk such as cow's milk, goat's milk, mare milk and sheep's milk, a dairy product such as skim milk powder, whole milk powder and fresh cream or the like and a synthetic medium. Of the media, skim milk powder is preferable.

Depending on the need, the medium may contain a carbohydrate which the lactic acid bacterium can assimilate. Examples of such a carbohydrate include glucose, sucrose, fructose and the like. The amount of the carbohydrate which the lactic acid bacterium can assimilate contained in the medium is not particularly limited and may be appropriately determined in accordance with the lactic acid bacterium used for the culture and the like, but the amount is, for example, 0.1 to 20%, preferably 1 to 10%.

According to the need, the medium may further contain an auxiliary culture agent such as a Chinese sweet tea extract, an oolong tea extract, a green tea extract, a black tea extract, a jasmine tea extract and oleic acid or the like. One kind of the auxiliary culture agents or a combination of two or more kinds thereof can be used.

Regarding the Chinese sweet tea extract, the oolong tea extract, the green tea extract, the black tea extract and the jasmine tea extract of the auxiliary culture agents, those which are commercially available and those obtained by the methods described in JP-A-2001-178413, JP-A-2016-174589 and the like may be used. The extract content of the medium is not particularly limited, but for example, the Chinese sweet tea extract or oolong tea extract content is 0.1 to 2%, preferably 0.22 to 0.33%.

Of the auxiliary culture agents, oleic acid or the like is not particularly limited, and in addition to free oleic acid and inorganic salts of oleic acid, sugar esters, glycerides, sorbitan esters, propylene glycol esters and the like which are generally used as emulsifiers and in which the fatty acid moiety is oleic acid can be used. A food material which contains a large amount of oleic acid or the like can also be used.

Specific examples of oleic acid or the like include salts of oleic acid such as sodium oleate and potassium oleate and oleate esters such as glyceryl oleate, polyglyceryl oleate and sucrose oleate. One or more kinds of oleic acid and the like can be used.

The amount of oleic acid or the like contained in the medium is not particularly limited but is, for example, 5 to 200 ppm, preferably 10 to 100 ppm.

The medium may further contain a component which can be added to a general medium that is used for culturing a lactic acid bacterium, such as vitamins including vitamin A, vitamin B's, vitamin C, vitamin E and the like, peptides including milk peptides and the like, amino acids, salts of calcium, magnesium and the like, saccharides and the like.

The medium, which contains the oligosaccharide, can be directly used for culturing the lactic acid bacterium without a problem, but the osmotic pressure thereof is preferably adjusted because the fermentability becomes excellent and because the bacterial count after culture and the bacterial count after preservation become both high. The osmotic pressure of the medium is 500 to 1400 mOsm, preferably 800 to 1200 mOsm. The method for adjusting the osmotic pressure is not particularly limited, and examples thereof include a method in which the amounts of the oligosaccharide and a saccharide contained in the medium are adjusted and another method. That the fermentability becomes excellent means that the acidity after the culture is 24 or more and that the bacterial count is 5.0×10⁹ cfu/ml or more.

A preferable embodiment of the medium containing the oligosaccharide used in the production method of the present invention is as follows.
Skim milk powder 10 to 20%
Oolong tea extract 0.1 to 2%
Oligosaccharide 1 to 20%
Glucose 0.1 to 20%

The lactic acid bacterium used in the production method of the present invention is not particularly limited and may be, for example, any of those found in the nature, those deposited at depositary authorities, those which are commercially available and mutants thereof. Specific examples thereof include lactic acid bacteria of the genus *Lactobacillus* such as *Lactobacillus casei, Lactobacillus gasseri, Lactobacillus acidophilus, Lactobacillus cremoris, Lactobacillus helveticus, Lactobacillus salivarius, Lactobacillus fermentum, Lactobacillus jugurti, Lactobacillus delbrueckii* subsp. *bulgaricus, Lactobacillus delbrueckii* subsp. *delbrueckii* and *Lactobacillus johnsonii;* lactic acid bacteria of the genus *Streptococcus* such as *Streptococcus thermophilus;* lactic acid bacteria of the genus *Lactococcus* such as *Lactococcus lactis* subsp. *lactis, Lactococcus lactis* subsp. *cremoris, Lactococcus plantarum* and *Lactococcus raffinolactis;* lactic acid bacteria of the genus *Enterococcus* such as *Enterococcus faecalis* and *Enterococcus faecium* and the like. Of the lactic acid bacteria, lactic acid bacteria which do not substantially assimilate oligosaccharides are preferable, and examples thereof include lactic acid bacteria such as *Lactobacillus casei, Lactobacillus gasseri, Lactobacillus acidophilus* and *Streptococcus thermophilus.* Here, that a lactic acid bacterium does not substantially assimilate oligosaccharides means that the lactic acid bacterium does not have the ability of assimilating oligosaccharides of tri- or higher saccharides. The assimilation ability can be examined by a known method, and examples thereof include a method of culturing the lactic acid bacterium in a medium containing oligosaccharides alone as the sugar source and then examining the presence or absence of colonies or the turbidity of the medium and another method. Of the lactic acid bacteria which do not substantially assimilate oligosaccharides, lactic acid bacteria of the genus *Lactobacillus* are preferable, and examples thereof include lactic acid bacteria such as *Lactobacillus casei.* In particular, of the lactic acid bacteria of the genus *Lactobacillus, Lactobacillus casei* YIT9029 (FERM BP-1366, deposition date of January 12, 1981) and the like are preferable. One kind of the lactic acid bacteria or a combination of two or more kinds thereof can be used.

The lactic acid bacterium which is described above with the deposition date has been deposited at International Patent Organism Depositary, National Institute of Technology and Evaluation (#120, 2-5-8 Kazusakamatari, Kisarazu-shi, Chiba 292-0818, Japan).

In the production method of the present invention, the conditions for culturing the lactic acid bacterium in a medium containing the oligosaccharide are not particularly limited and may be appropriately determined according to the kind of the lactic acid bacterium used and the like.

Specifically, when the lactic acid bacterium is cultured in a medium containing the oligosaccharide, the conditions are that the lactic acid bacterium is inoculated in such a manner that the bacterial count of the lactic acid bacterium in the medium becomes around 0.5×10⁶ cfu/ml and that the lactic acid bacterium is cultured at the optimum culture temperature of the lactic acid bacterium or the like or at another temperature until the pH becomes around 3.5 to 3.6 or until the acidity becomes around 24.0. Regarding the culturing conditions here, a method which is suitable for culturing the lactic acid bacterium used may be appropriately selected from static method, stirring, shaking, aeration and the like.

The fermented milk containing the oligosaccharide and the lactic acid bacterium as a living bacterium obtained after the culture may be directly used as a final product, but an oligosaccharide may be further added. The amount of the oligosaccharide added in this case is not particularly limited but is 1 to 20%, preferably 3 to 10% relative to the final product.

In the fermented milk, a food material such as a carbohydrate, a sugar alcohol, a high-intensity sweetener, a thickener, an emulsifier, a vitamin and a flavor can be further blended by a conventionally known method depending on the need.

The food materials are carbohydrates such as sucrose, glucose, fructose, palatinose, trehalose, lactose, xylose and maltose; sugar alcohols such as sorbitol, xylitol, erythritol, lactitol, Palatinit, reduced starch syrup and reduced maltose syrup; high-intensity sweeteners such as aspartame, thaumatin, sucralose, acesulfame K and stevia; thickeners (stabilizers) such as agar, gelatin, carrageenan, guar gum, xanthan gum, pectin, locust bean gum, gellan gum, carboxymethylcellulose, soybean polysaccharides and propylene glycol alginate; emulsifiers such as sucrose fatty acid esters, glycerol fatty acid esters, polyglycerol fatty acid esters, sorbitan fatty acid esters and lecithin; milk fat such as cream, butter and sour cream; acidulants such as citric acid, lactic acid, acetic acid, malic acid, tartaric acid and gluconic acid; vitamins such as vitamin A, vitamin B's, vitamin C and vitamin E's; minerals such as calcium, magnesium, zinc, iron and manganese and flavors such as yogurt flavor, berry flavor, orange flavor, Chinese quince flavor, perilla flavor, citrus flavor, apple flavor, mint flavor, grape flavor, apricot flavor, pear, custard cream, peach, melon, banana, tropical flavor, herbal flavor, black tea and coffee flavor.

The fermented milk containing the oligosaccharide and the lactic acid bacterium as a living bacterium explained above includes not only fermented milk specified by Ministerial Ordinance Concerning Compositional Standards, etc. for Milk and Milk Products but also beverages such as dairy lactic acid bacteria beverages and lactic acid bacteria beverages and those containing living bacteria such as kefir and yogurt. Examples of the form thereof include hard type, soft type, plain type, sweetened type, fruit type, drink type, frozen type and the like.

The fermented milk has improved viability of the lactic acid bacterium and can maintain a high viable bacterial count during long-term preservation. Specifically, when the fermented milk is preserved at 10°C or lower for 25 days, the viability is 95% or more. The viability is a value measured by the method described in the Examples.

The fermented milk has an enhanced healthy image. The flavor of fermented milk having a healthy image means a flavor which can give a low-calorie, low-carbohydrate and fermented milk-like image due to no excessive sweetness and due to moderate sourness generated with fermentation of the lactic acid bacterium.

### Examples

The present invention is explained in detail below referring to Examples, but the present invention is not limited to the Examples.

### Reference Example 1

### Production of Conventional Fermented Milk:

A medium obtained by dissolving 14.7% skim milk powder, 9.2% high-fructose corn syrup and 0.14% oolong tea extract (YOE-N: manufactured by Yakult Material Co., Ltd.) in water was sterilized at 100°C for 60 minutes. A starter of *Lactobacillus casei* YIT9029 (FERM BP-1366) was inoculated at 0.5% (initial bacterial count: 5.0×10⁶ cfu/ml) to the medium and cultured while the temperature was adjusted until the acidity became 24.3. The obtained fermented milk was mixed with a stabilizer solution containing 2.9% skim milk powder and 2.7% soybean polysaccharides (manufactured by Fuji Oil Co., Ltd., SM-YT) as a stabilizer, homogenized and then mixed with sucrose syrup or galactooligosaccharide syrup (a mixture of 56.4% galactooligosaccharide and 43% other saccharides based on the solid content: Oligomate 55N: manufactured by Yakult Pharmaceutical Industry Co., Ltd.) shown in Table 1, and lactic acid bacteria beverages having a viable bacterial count of 1.4×10⁹ cfu/ml, an acidity of 5.8 and pH of 3.7) were thus obtained (products 1 and 2). The lactic acid bacteria beverages were put into containers and preserved at 10°C, and then the viabilities were measured by the following method. The results are shown in Table 1.

### <Method for Measuring Acidity>

The titer (unit: ml/9 g) derived by adding 40 g of ion-exchanged water to 9 g of the culture and conducting neutralization titration with 0.1N sodium hydroxide until the pH became 8.5 was regarded as the acidity.

### <Method for Measuring Osmotic Pressure>

Measurement was conducted using an osmometer "Osmo Station OM-6060" manufactured by ARKRAY Inc.

### <Method for Measuring Viable Bacterial Count>

Measurement was conducted using BCP medium (Eiken Chemical Co., Ltd.).

### <Method for Calculating Viability>

viability (%) on day X of preservation = (viable bacterial count on day X of preservation/viable bacterial count on day 0 of preservation) × 100

**[Table 1]**

| | Product 1 | Product 2 |
|---|---|---|
| Saccharides added to medium | 9.2% high-fructose corn syrup | 9.2% high-fructose corn syrup |
| Osmotic pressure of medium | 1117 mOsm | 1117 mOsm |
| Syrup | 21.35% sucrose | 18.3% galactooligosaccharide syrup |
| Viability (25-day preservation) | 99% | 92% |

When the galactooligosaccharide was added as syrup to the fermented milk after fermentation, the viability was lower than that obtained when sucrose was added as syrup to the fermented milk after fermentation.

### Example 1

### Production of Fermented Milk:

A medium obtained by dissolving 14.6% skim milk powder, 0.14% oolong tea extract (YOE-N: manufactured by Yakult Material Co., Ltd.) and 19.5% galactooligosaccharide syrup (a mixture of 56.4% galactooligosaccharide and 43% other saccharides based on the solid content: Oligomate 55N: manufactured by Yakult Pharmaceutical Industry Co., Ltd.) in water was sterilized at 100°C for 60 minutes. A starter of *Lactobacillus casei* YIT9029 (FERM BP-1366) was inoculated at 0.5% (initial bacterial count: 5.0×10⁶ cfu/ml) to the medium and cultured while the temperature was adjusted until the acidity became 24.3. The obtained fermented milk was mixed with a stabilizer solution containing 2.9% skim milk powder and 2.7% soybean polysaccharides (manufactured by Fuji Oil Co., Ltd., SM-YT) as a stabilizer, homogenized and then mixed into syrup containing 16.1% galactooligosaccharide syrup shown in Table 2, and a lactic acid bacteria beverage having a viable bacterial count of 6.3×10⁸ cfu/ml, a titration acidity of 4.9 ml/9 g and pH of 3.8 was thus obtained (Example product 1). The lactic acid bacteria beverage was put into a container and preserved at 10°C, and then the viability was measured by the above method. The results are shown in Table 2. In this regard, the object of comparison was product 2 produced in Reference Example 1.

**[Table 2]**

| | Product 2 | Example product 1 |
|---|---|---|
| Saccharides added to medium | 9.2% high-fructose corn syrup | 19.5% galactooligosaccharide syrup (containing 8.2% galactooligosaccharide and 3.4% or more glucose) |
| Osmotic pressure of medium | 1117 mOsm | 1533 mOsm |
| Syrup | 18.3% galactooligosaccharide syrup | 16.1% galactooligosaccharide syrup |
| Viability (25-day preservation) | 92% | 101% |

When the galactooligosaccharide was added to the medium, the viability was significantly higher than that obtained when the galactooligosaccharide was added as syrup to the fermented milk after fermentation.

### Example 2

### Production of Fermented Milk:

A medium obtained by dissolving 14.6% skim milk powder, 0.14% oolong tea extract (YOE-N: manufactured by Yakult Material Co., Ltd.) and 12.8% galactooligosaccharide syrup (a mixture of 56.4% galactooligosaccharide and 43% other saccharides based on the solid content: Oligomate 55N: manufactured by Yakult Pharmaceutical Industry Co., Ltd.) in water was sterilized at 100°C for 60 minutes. A starter of *Lactobacillus casei* YIT9029 (FERM BP-1366) was inoculated at 0.5% (initial bacterial count: 5.0×10 cfu/ml) to the medium and cultured while the temperature was adjusted until the acidity became 24.3. The obtained fermented milk was mixed with a stabilizer solution containing 2.9% skim milk powder and 2.7% soybean polysaccharides (manufactured by Fuji Oil Co., Ltd., SM-YT) as a stabilizer, homogenized and then mixed into syrup containing 18.3% galactooligosaccharide syrup shown in Table 3, and a lactic acid bacteria beverage having a viable bacterial count of 1.2×10⁹ cfu/ml, a titration acidity of 5.6 and pH of 3.7 was thus obtained (Example product 2). The lactic acid bacteria beverage was put into a container and preserved at 10°C, and then the viable bacterial count (and the viability) was measured by the above method. The results are shown in Table 3. In this regard, the objects of comparison were product 3 obtained by adding 2.3% glucose and 0.014% sucralose to the medium instead of the galactooligosaccharide and product 4 obtained by adding 2.3% glucose and 10.8% maltitol instead of the galactooligosaccharide. The fermentabilities of the products were assessed by the following assessment criteria. The results are also shown in Table 3.

### <Assessment Criteria for Fermentability>

### (Assessment) (Contents)

A: The acidity after the culture is 24 or more, and the viable bacterial count is 5.0×10⁹ cfu/ml or more.
B: The acidity after the culture is 24 or more, and the viable bacterial count is less than 5.0×10⁹ cfu/ml.
C: The acidity after the culture is less than 24.

**[Table 3]**

| | Example product 1 | Example product 2 | Product 3 | Product 4 |
|---|---|---|---|---|
| Saccharides added to medium | 19.5% galactooligosaccharide syrup (containing 8.2% galactooligosaccharide and 3.4% or more glucose) | 12.8% galactooligosaccharide syrup (containing 5.3% galactooligosaccharide and 2.2% or more glucose) | 2.3% glucose + 0.014% sucralose | 2.3% glucose + 10.8% maltitol |
| Osmotic pressure of medium | 1533 mOsm | 1131 mOsm | 652 mOsm | 1254 mOsm |
| Syrup | 16.1% galactooligosaccharide syrup | 18.3% galactooligosaccharide syrup | 18.3% galactooligosaccharide syrup | 18.3% galactooligosaccharide syrup |
| Fermentability | B | A | C* | C* |
| Acidity after culture | 24.4 | 24.5 | 23.5 | 23.2 |
| Viable bacterial count after culture in medium* | 2.8×10⁹ | 5.3×10⁹ | 9.7×10⁸ | 4.9×10⁹ |
| Viable bacterial count^{∗} (25-day preservation) | 6.4×10⁸ | 1.2×10⁹ | 1.6×10⁸ | 9.8×10⁸ |
| Viability (25-day preservation) | 101% | 103% | 76% | 98% |

| | | | | |
|---|---|---|---|---|
| * The unit of bacterial count is cfu/ml. * The acidity did not reach the aimed value. | | | | |

It was found that, by adjusting the osmotic pressure of the medium between 800 and 1200 mOsm, the fermentability becomes excellent, and the bacterial count after culture and the bacterial count after preservation both become high (Example product 2). Moreover, when sucralose, maltitol or the like, which are not galactooligosaccharides, was added to the medium, the acidity after the culture did not reach the aimed value whereby it is considered that the fermentability was adversely affected, and the acidity of the product did not reach the aimed value (products 3 and 4) .

### Example 3

### Production of Fermented Milk:

A medium obtained by dissolving 14.6% skim milk powder, 0.14% oolong tea extract (YOE-N: manufactured by Yakult Material Co., Ltd.) and 12.8% galactooligosaccharide syrup (a mixture of 56.4% galactooligosaccharide and 43% other saccharides based on the solid content: Oligomate 55N: manufactured by Yakult Pharmaceutical Industry Co., Ltd.) in water was sterilized at 100°C for 60 minutes. A starter of *Lactobacillus casei* YIT9029 (FERM BP-1366) was inoculated at 0.5% (initial bacterial count: 5.0×10⁶ cfu/ml) to the medium and cultured while the temperature was adjusted until the acidity became 24.3. The obtained fermented milk was mixed with a stabilizer solution containing 2.9% skim milk powder and 2.7% soybean polysaccharides (manufactured by Fuji Oil Co., Ltd., SM-YT) as a stabilizer, homogenized and then mixed into syrup containing 0.02% stevia and 18.3% galactooligosaccharide syrup shown in Table 4, and a lactic acid bacteria beverage having a viable bacterial count of 1.2×10⁹ cfu/ml, a titration acidity of 5.2 and pH of 3.8 was thus obtained (Example product 3). The lactic acid bacteria beverage was put into a container and preserved at 10°C, and then the viable bacterial count (and the viability) was measured by the above method. The results are shown in Table 4. The object of comparison was product 5 obtained by adding 9.2% high-fructose corn syrup to the medium instead of the galactooligosaccharide. With respect to Example product 3 and product 5, the acidities of the products were both adjusted to 5.2, and the relative degrees of sweetness were both adjusted to 110. Here, the relative degree of sweetness is the relative value when the degree of sweetness of aqueous 10 w/v% sucrose solution is regarded as 100. The sweetness, the sourness and the healthy images of the products were assessed by the following assessment criteria, and the flavors were assessed by free description. The results are also shown in Table 4.

### <Assessment Criteria for Sweetness and Sourness>

| (Score) | (Contents) |
|---|---|
| 1: | weak |
| 2: | slightly weak |
| 3: | moderate |
| 4: | slightly strong |
| 5: | strong |

### <Assessment Criteria for Healthy Image>

| (Score) | (Contents) |
|---|---|
| 1: | without healthy image |
| 2: | without clear healthy image |
| 3: | neither with nor without healthy image |
| 4: | with slight healthy image |
| 5: | with healthy image |

**[Table 4]**

| | Example product 3 | Product 5 |
|---|---|---|
| Saccharides added to medium | 12.8% galactooligosaccharide syrup (containing 5.3% galactooligosaccharide and 2.2% or more glucose) | 9.2% high-fructose corn syrup |
| Osmotic pressure of medium | 1131 mOsm | 1088 mOsm |
| Syrup | 18.3% galactooligosaccharide syrup, 0.02% stevia | 18.3% galactooligosaccharide syrup, 0.02% stevia |
| Strength of sweetness | 3 | 4 |
| Strength of sourness | 3 | 3 |
| Healthy image | 5 | 3 |
| Free description | Clear, good balance of sourness and sweetness | Intense sweetness, slightly heavy |

In general, when the sourness of fermented milk becomes relatively stronger than the sweetness, the palatability decreases, while too strong sweetness rather impairs the clear, fresh taste and the healthy flavor image required for fermented milk and lactic acid bacteria beverages. Whereas the intense sweetness of high-fructose corn syrup of product 5 rather leads to a high-calorie image and impairs the healthy image, Example product 3 does not have excessive sweetness but has clear taste with a good balance with the sourness, which leads to a healthy image, because the galactooligosaccharide syrup was the only raw saccharide material.

### Industrial Applicability

The present invention can be used for producing fermented milk containing an oligosaccharide and a lactic acid bacterium as a living bacterium which can maintain a high viable bacterial count even during long-term preservation.

## Claims

1. A method for producing fermented milk containing an oligosaccharide and a lactic acid bacterium as a living bacterium which is **characterized by** culturing the lactic acid bacterium in a medium containing the oligosaccharide.

2. The method for producing fermented milk according to claim 1, wherein the osmotic pressure of the medium during the culture is 800 to 1200 mOsm.

3. The method for producing fermented milk according to claim 1 or 2, wherein the oligosaccharide is a galactooligosaccharide.

4. The method for producing fermented milk according to any of claims 1 to 3, wherein the lactic acid bacterium is a lactic acid bacterium which does not substantially assimilate oligosaccharides.

5. The method for producing fermented milk according to any of claims 1 to 4, wherein the lactic acid bacterium is a lactic acid bacterium of the genus *Lactobacillus.*

6. The production method according to any of claims 1 to 5 in which an oligosaccharide is further added after culturing the lactic acid bacterium in the medium containing the oligosaccharide.

7. The method for producing fermented milk according to any of claims 1 to 6, wherein the amount of the oligosaccharide in the medium is 5.0 mass% or more.

8. Fermented milk produced by the method for producing fermented milk according to any of claims 1 to 7.

9. A method for improving the viability of a lactic acid bacterium in fermented milk containing an oligosaccharide and the lactic acid bacterium as a living bacterium which is **characterized by** culturing the lactic acid bacterium in a medium containing the oligosaccharide.
